Europäisches Patentamt

European Patent Office

Office européen des brevets

(19)

(11) Veröffentlichungsnummer: **0 499 080 A2**

# (12) EUROPÄISCHE PATENTANMELDUNG

(21) Anmeldenummer: **92101268.8**

(22) Anmeldetag: **27.01.92**

(51) Int. Cl.⁵: **C07D 295/215**, **A61K 31/445**

(30) Priorität: **09.02.91 DE 4104024**

(43) Veröffentlichungstag der Anmeldung:
**19.08.92 Patentblatt 92/34**

(84) Benannte Vertragsstaaten:
**AT BE CH DE DK ES FR GB GR IT LI LU NL SE**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Raddatz, Peter, Dr.**
**Akazienweg 8a**
**W-6104 Seeheim(DE)**
Erfinder: **Schmitges, Claus J., Dr.**
**Karolinger Strasse 5**
**W-6114 Gross-Umstadt(DE)**
Erfinder: **Minck, Klaus-Otto, Dr.**
**Büchestrasse 8**
**W-6105 Ober-Ramstadt(DE)**

(54) **Aminosäurederivate.**

(57) Neue Aminosäurederivate der Formel I

$$X\text{-}W\text{-}CR^1R^2\text{-}CO\text{-}NH\text{-}CH_2CH_2\text{-}CO\text{-}NH\text{-}CHR^3\text{-}(CHOH)_2\text{-}R^4 \quad I$$

worin $R^1$ bis $R^4$, X und W die in Patentanspruch 1 angegebenen Bedeutungen haben,
sowie ihre Salze hemmen die Aktivität des menschlichen Plasmarenins.

EP 0 499 080 A2

Die Erfindung betrifft neue Aminosäurederivate der Formel I

X-W-CR¹R²-CO-NH-CH₂CH₂-CO-NH-CHR³-(CHOH)₂-R⁴   I

worin

X
H, $R^5$-O-$C_mH_{2m}$-CO-, $R^5$-$C_mH_{2m}$-O-CO-, $R^5$-$C_mH_{2m}$-CO-, $R^5$-SO₂-, $R^6R^7$N-$C_mH_{2m}$-CO-, $R^6$-NH-C(=NH)-NH-$C_mH_{2m}$-CO-, $R^6$OOC-$C_mH_{2m}$-CO-, $R^6O_3$S-$C_mH_{2m}$-CO-, $R^6$-O-(CH₂CH₂O)$_n$-$C_mH_{2m}$-CO-, $A_3N^+$-$C_mH_{2m}$-CO- An⁻ oder $R^6$OOC-CH-(NR⁸R⁹)-$C_pH_{2p}$-Y-CO-,

W und Y
jeweils CH₂, O oder NH,

R¹, R⁷ und R⁸
jeweils H oder A,

R², R³ und R⁵
jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

R⁴
H, A, Ar-alkyl, Cycloalkyl mit 3-7 C-Atomen oder Cycloalkylalkyl mit 4-11 C-Atomen,

R⁶ und R⁹
jeweils H, A oder $R^5$-$C_pH_{2p}$-Y-CO-,

R⁶R⁷N
auch eine unsubstituierte oder eine durch A, OH, NH₂, NHA, NA₂, NHAc, NH-CO-$C_xH_{2x}$-O-R¹⁰, NH-CO-O-$C_xH_{2x}$-R¹⁰, Hydroxy-alkyl, COOH, COOA, CONH₂, Amino-alkyl, HAN-alkyl, A₂N-alkyl, $A_3N^\oplus$alkyl An⁻, NH-CO-NH₂, NH-CO-NHA, Guanidinyl oder Guanidinyl-alkyl substituierte Pyrrolidino-, Piperidino-, Morpholino- oder Piperazino-gruppe,

R¹⁰
H, A, Ar-alkyl oder CN,

m, p und x
jeweils 0, 1, 2, 3, 4, 5, 6, 7, 8, 9 oder 10,

n
0, 1, 2 oder 3,

Ar
unsubstituiertes oder ein- oder mehrfach durch A, OA, Hal, CF₃, OH, NO₂, Hydroxy-alkyl, NH₂, NHA, NA₂, NHAc, NH-SO₂-A, SA, SO-A, SO₂-A, SO₂NH₂, SO₂NHA, COOH, COOA, CONH₂, CN, Amino-alkyl, HAN-alkyl, A₂N-alkyl, $A_3N^\oplus$-alkyl An⁻ und/oder Guanidinyl-alkyl substituiertes Phenyl oder unsubstituiertes Naphthyl,

Het
einen gesättigten oder ungesättigten 5-oder 6-gliedrigen heterocyclischen Rest mit 1-4 N-, O- und/oder S-Atomen, der mit einem Benzolring kondensiert und/oder ein- oder mehrfach durch A, OA, Hal, CF₃, OH, NO₂, Carbonylsauerstoff, NH₂, NHA, NA₂, NHAc, NH-COOA, NHCOOAr, NHCOOCH₂Ar, NH-SO₂-A, SA, SO-A, SO₂-A, SO₂NH₂, SO₂NHA, COOH, COOA, CONH₂, CN, Ar, Ar-alkyl, Ar-alkenyl, Hydroxy-alkyl, Amino-alkyl, HAN-alkyl, A₂N-alkyl und/oder $A_3N^\oplus$-alkyl An⁻ substituiert sein kann und/oder dessen N- und/oder S-Heteroatome auch oxydiert sein können,

Hal
F, Cl, Br oder J,

Ac
A-CO-, Ar-CO-, Ar-alkyl-CO- oder A-NH-CO-,

An⁻
ein Anion, das auch fehlen kann, wenn stattdessen eine in der Verbindung der Formel I enthaltene Carboxygruppe in Form eines Carboxylatanions vorliegt,

-alkyl
eine Alkylengruppe mit 1-8 C-Atomen und

A
Alkyl mit 1-8 C-Atomen bedeuten,
worin ferner an Stelle einer oder mehrerer -NH-CO-Gruppen auch eine oder mehrere -NA-CO-Gruppen stehen können,
sowie deren Salze.

Ähnliche Verbindungen sind aus der EP-A-332008 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B. nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z.B. Pepsin und Kathepsin D) sind in der Regel etwa 100 bis 1000mal so hohe Konzentrationen dieser Verbindungen notwendig wie für die Renin-Hemmung. Die Wirkungen der Verbindungen auf den Blutdruck und/oder auf die Herzfrequenz sowie die Hemmung der Reninaktivität im Blutplasma können ferner an wachen Affen, z.B. weiblichen Affen (Macaca fascicularis) ermittelt werden; dabei können Blutdruck und Herzfrequenz in Anlehnung an die Methode von M.J. Wood et al., J. Hypertension 4, 251-254 (1985) gemessen werden.

Zur Stimulierung der Reninaktivität werden die Tiere dabei zweckmäßig mit einem Saluretikum vorbehandelt. Blutproben zur Bestimmung der Plasma-Reninaktivität können durch Punktion der Vena femoralis gewonnen werden.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können ausgeführt werden ähnlich wie es in der EP-A-77 028 angegeben ist.

Die vor- und nachstehend aufgeführten Abkürzungen von Aminosäureresten stehen für die Reste -NR'-R''-CO-, in der Regel -NH-CHR-CO- (worin R, R' und R'' die für jede Aminosäure bekannte spezifische Bedeutung haben) folgender Aminosäuren:

| Ada | 3-(1-Adamantyl)-alanin |
| Ala | Alanin |
| $\beta$Ala | $\beta$-Alanin |
| Bia | 3-(2-Benzimidazolyl)-alanin |
| Cal | 3-Cyclohexylalanin |
| Gly | Glycin |
| His | Histidin |
| Hph | Homophenylalanin (2-Amino-4-phenyl-buttersäure) |
| Ile | Isoleucin |
| Leu | Leucin |
| Mal | 3-(p-Methoxyphenyl)-alanin |
| $\alpha$Nal | 3-($\alpha$-Naphthyl)-alanin |
| $\beta$Nal | 3-($\beta$-Naphthyl)-alanin |
| Nle | Norleucin |
| Nva | Norvalin |
| Phe | Phenylalanin |
| Tia | 3-(Thienyl)-alanin [z.B. 2-Tia = 3-(2-Thienyl)-alanin] |
| Tiz | 3-(Thiazolyl)-alanin [z.B. 2-Tiz = 3-(2-Thiazolyl)-alanin] |
| Trp | Tryptophan |
| Tyr | Tyrosin. |

Ferner bedeutet nachstehend:

| BOC | tert.-Butoxycarbonyl |
| BOM | Benzyloxymethyl |
| imi-BOM | Benzyloxymethyl in 1-Stellung des Imidazolrings |
| CBZ | Benzyloxycarbonyl |
| DCCI | Dicyclohexylcarbodiimid |
| DMF | Dimethylformamid |
| DNP | 2,4-Dinitrophenyl |
| imi-DNP | 2,4-Dinitrophenyl in 1-Stellung des Imidazolrings |
| ETOC | Ethoxycarbonyl |
| FMOC | 9-Fluorenylmethoxycarbonyl |
| HOBt | 1-Hydroxybenzotriazol |
| IPOC | Isopropoxycarbonyl |
| Pla | den Rest der Phenylmilchsäure -O-CH($CH_2 C_6 H_5$)-CO- (S-Form) |

| POA | Phenoxyacetyl |
| THF | Tetrahydrofuran. |

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Carbonsäure der Formel II

X-G$^1$-OH    II

worin

G$^1$

(a) fehlt,
(b) -W-CR$^1$R$^2$-CO-,
(c) -W-CR$^1$R$^2$-CO-NH-CH$_2$CH$_2$-CO-
bedeutet

oder eines ihrer reaktionsfähigen Derivate mit einer Verbindung der Formel III

H-G$^2$-NH-CHR$^3$-(CHOH)$_2$-R$^4$    III

worin

G$^2$

(a) -W-CR$^1$R$^2$-CO-NH-CH$_2$CH$_2$-CO-,
(b) -NH-CH$_2$CH$_2$-CO- bedeutet,
(c) fehlt

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe durch Behandeln mit einem acylierenden Mittel acyliert und/oder eine Verbindung der Formel I durch Behandeln mit einer Säure in eines ihrer Salze überführt.

Vor- und nachstehend haben die Reste bzw. Parameter R$^1$ bis R$^{10}$, W, X, Y, m, n, p, x, Ar, Het, Hal, Ac, An, A, G$^1$ und G$^2$ die bei den Formeln I, II oder III angegebenen Bedeutungen, falls nicht ausdrücklich etwas anderes angegeben ist.

In den vorstehenden Formeln hat A 1-8, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B. 1-, 2- oder 3-Methylcyclopentyl, 1-, 2-, 3- oder 4-Methylcyclohexyl.

Dementsprechend bedeutet Cycloalkylalkyl vorzugsweise Cyclopropylmethyl, 2-Cyclopropy-lethyl, Cyclobutylmethyl, 2-Cyclobutylethyl, Cyclo-pentylmethyl, 2-Cyclopentylethyl, Cyclohexylme-thyl, 2-Cyclohexylethyl, aber auch z.B. 1-, 2- oder oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO-, wie Acetyl, Propionyl oder Butyryl, Ar-CO- wie Benzoyl, o-, m-oder p-Methoxybenzoyl oder 3,4-Dimethoxybenzoyl, A-NH-CO- wie N-Methyl-oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m-oder p-Hydroxyphenyl, o-, m- oder p-Sulfamoylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, o-, m-oder p-Aminomethylphenyl, o-, m- oder p-Dimethylaminomethylphenyl, o-, m- oder p-Guanidinomethylphenyl, 1- oder 2-Naphthyl.

Dementsprechend bedeutet Ar-alkyl vorzugsweise Benzyl, 1- oder 2-Phenylethyl, o-, m- oder p-Methylbenzyl, 1-oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1-oder 2-o-, -m- oder -p-Chlorphenylethyl, o-, m-oder p-Brombenzyl, 1- oder 2-o-, -m- oder -p-Bromphenylethyl, o-, m- oder p-Jodbenzyl, 1- oder 2-o-, -m-oder -p-Jodphenylethyl, o-, m- oder p-Trifluormethylbenzyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, o-, m- oder p-Aminomethylbenzyl, o-, m- oder p-Dimethylaminomethylbenzyl, o-, m- oder p-Guanidinomethylbenzyl, 1- oder 2-Naphthylmethyl. Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3-oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7-oder 8-Cinnolyl, 2-, 4-, 5-, 6- 7- oder 8-Chinazolyl. Die heterocyclischen Reste können auch teilweise oder vollständig hydriert sein. Het kann also z.B. auch bedeuten 2,3-Dihydro-2-, -3-, -4- oder -5-furyl, 2,5-Dihydro-2-, -3-, -4- oder -5-furyl, Tetrahydro-2-oder -3-furyl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5- oder -6-pyridyl, 1,2,3,6-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexa hydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann z.B. bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 4-Amino-2-methyl-5-pyrimidinyl, 2-Amino-5,6-dimethyl-3-pyrazinyl, 4-Carbamoylpiperidino, ferner z.B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 5-Methyl-3-isoxazolyl, 3,4-Dimethyl-5-isoxazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl,2- oder 3-Methyl-4-pyridyl, 3-, 4-, 5- oder 6-Chlor-2-pyridyl, 2-, 4-, 5- oder 6-Chlor-3-pyridyl, 2- oder 3-Chlor-4-pyridyl, 2,6-Dichlorpyridyl, 2-Hydrozy-3-, -4-, -5- oder -6-pyridyl (= 1H-2-Pyridon-3-, -4-, -5- oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 2,6-Dihydroxy-4-pyrimidinyl, 5-Chlor-2-methyl-4-pyrimidinyl, 3-Methyl-2-benzofuryl, 2-Ethyl-3-benzofuryl, 7-Methyl-2-benzothienyl, 1-, 2-, 4-, 5-, 6-oder 7-Methyl-3-indolyl, 1-Methyl-5- oder -6-benzimidazolyl, 1-Ethyl-5- oder -6-benzimidazolyl, 3-, 4-, 5-, 6-, 7- oder 8-Hydroxy-2-chinolyl, 2-Oxo-pyrrolidino, 2-Oxo-piperidino, 2,5-Dioxopyrrolidino, 3-Benzyl-2,5-dioxopyrrolidino.

X ist allgemein vorzugsweise H; $R^5$-$C_mH_{2m}$-O-CO- wie BOC; $R^5$-$C_mH_{2m}$-CO- wie Formyl oder Acetyl; $R^5$-$SO_2$-, insbesondere A-$SO_2$- wie Methylsulfonyl; $R^6R^7N$-$C_mH_{2m}$-CO-, insbesondere 4-BOC-amino-piperidinocarbonyl, 4-Amino-piperidinocarbonyl, 4-Hydroxy-piperidinocarbonyl, 4-Dimethylamino-piperidinocarbonyl, 4-Ethoxycarbonylamino-piperidinocarbonyl, Morpholinocarbonyl, Piperazinocarbonyl oder 4-BOC-piperazinocarbonyl.

Die Gruppen W und Y bedeuten jeweils vorzugsweise NH. $R^1$, $R^6$, $R^7$, $R^8$ und $R^9$ bedeuten jeweils bevorzugt H, ferner bevorzugt Methyl. $R^6R^7N$ ist bevorzugt auch Pyrrolidino, Piperidino, Morpholino, Amino-piperidino wie 4-Amino-piperidino, Hydroxypiperidino wie 4-Hydroxypiperidino, Alkylaminopiperidino wie 4-Methylaminopiperidino, Dialkyl-aminopiperidino wie 4-Dimethylaminopiperidino, Ethoxycarbonylamino-piperidino wie 4-Ethoxycarbonylamino-piperidino, BOC-amino-piperidino wie 4-BOC-amino-piperidino, Morpholino oder 4-BOC-piperazino.

$R^2$ ist vorzugsweise Ar-alkyl, insbesondere Benzyl oder p-Methoxybenzyl; ferner bevorzugt A, insbesondere n-Butyl oder Isobutyl; Cycloalkyl-alkyl, insbesondere Cyclohexylmethyl; Het-alkyl, insbesondere 2-Thienylmethyl. Die Gruppe -W-$CR^1R^2$-CO- bedeutet bevorzugt einen der Reste Phe oder Pla, ferner Ada, Bia, Cal, His, Hph, Ile, Leu, Mal, αNal, βNal, Nle, Nva, Tia, Tiz, Trp oder Tyr.

$R^3$ ist vorzugsweise Cycloalkylalkyl, insbesondere Cyclohexylmethyl, ferner bevorzugt Alkyl, insbesondere n-Butyl oder Isobutyl; Ar-alkyl, insbesondere Benzyl oder p-Methoxybenzyl; Het-alkyl, z. B. 2-Thienylmethyl; Cycloalkyl, insbesondere Cyclohexyl.

$R^4$ ist vorzugsweise A, z.B. Ethyl, Propyl, Isopropyl, Butyl oder Isobutyl, oder Cycloalkyl, z.B. Cyclopropyl, ferner Cyclobutyl oder Cyclopentyl.

$R^{10}$ ist vorzugsweise H, Methyl oder CN.

Der Parameter m ist vorzugsweise 1, 2, 3, 4 oder 5; p ist vorzugsweise 2 oder 3; n ist vorzugsweise 1; x ist vorzugsweise 1 oder 2.

$C_mH_{2m}$ und $C_xH_{2x}$ sind vorzugsweise geradkettig, bedeuten also vorzugsweise -$(CH_2)_m$- oder -$(CH_2)_x$-.

Dementsprechend bedeutet die Gruppe X im einzelnen vorzugsweise H; $R^7R^8N$-$(CH_2)_m$-CO-, vor allem $H_2N$-$C_mH_{2m}$-CO- wie Aminocarbonyl, Aminoacetyl (H-Gly-), 3-Aminopropionyl (H-βAla-), 4-Aminobutyryl, 5-Aminopentanoyl, 6-Aminohexanoyl, 7-Aminoheptanoyl, 8-Aminooctanoyl, 9-Aminononanoyl, 10-Aminodecanoyl, 11-Aminoundecanoyl, aber auch z.B. 2-Amino-propionyl (Ala), 2-Amino-2-methyl-propionyl, 3-Amino-3-methyl-butyryl; ANH-$C_mH_{2m}$-CO- wie Methylaminocarbonyl, Ethylaminocarbonyl, Methylaminoacetyl (Sarcosyl), 3-Methylaminopropionyl, 4-Methylaminobutyryl, 5-Methylaminopentanoyl, 6-Methylaminohexanoyl, 6-Ethylaminohexanoyl, 7-Methylaminoheptanoyl, 8-Methylaminooctanoyl, 9-Methylaminononanoyl, 10-Methylaminodecanoyl, 11-Methylaminoundecanoyl; $A_2N$-$C_mH_{2m}$-CO- wie Dimethylaminocarbonyl, Dimethylaminoacetyl, 3-Dimethylaminopropionyl, 4-Dimethylaminobutyryl, 5-Dimethylaminopentanoyl, 6-Dimethylaminohexanoyl, 6-Diethylaminohexanoyl, 7-Dimethylaminoheptanoyl, 8-Dimethylaminooctanoyl, 9-Dimethylaminononanoyl, 10-Dimethylaminodecanoyl, 11-Dimethylaminoundecanoyl; Pyrrolidino-$C_mH_{2m}$-CO- wie Pyrrolidinocarbonyl, Pyrrolidinoacetyl, 3-Pyrrolidino-propionyl, 4-Pyrrolidinobutyryl, 5-Pyrrolidino-pentanoyl, 6-Pyrrolidino-hexanoyl, 7-Pyrrolidino-heptanoyl, 8-Pyrrolidino-octanoyl, 9-Pyrrolidino-nonanoyl, 10-Pyrrolidino-decanoyl; Piperidino-$C_mH_{2m}$-CO-wie Piperidino-carbonyl, Piperidinoacetyl, 3-Piperidino-propionyl, 4-Piperidino-butyryl, 5-Piperidino-pentanoyl, 6-Piperidino-hexanoyl, 7-Piperidinoheptanoyl, 8-Piperidino-octanoyl, 9-Piperidino-nonanoyl, 10-Piperidino-decanoyl; Morpholino-$C_mH_{2m}$-CO- wie Morpholinocarbonyl, Morpholinoacetyl, 3-Morpholino-propionyl, 4-Morpholino-butyryl, 5-Morpholinopentanoyl, 6-Morpholino-hexanoyl, 7-Morpholino-heptanoyl, 8-Morpholino-octanoyl, 9-Morpholino-nonanoyl, 10-Morpholino-decanoyl; 4-Hydroxy-piperidino-$C_mH_{2m}$-CO- wie 4-Hydroxypiperidinocarbonyl, 4-Hydroxypiperidino-acetyl; 4-Amino-piperidino-$C_mH_{2m}$-CO- wie 4-Amino-piperidino-carbonyl, 4-Amino-piperidino-acetyl, 3-(4-Amino-piperidino)-propionyl, 4-(4-Amino-piperidino)-butyryl, 5-(4-Amino-piperidino)-pentanoyl, 6-(4-Amino-piperidino)-hexanoyl, 7-(4-Amino-piperidino)-heptanoyl, 8-(4-Amino-piperidino)-octanoyl, 9-(4-Amino-piperidino)-nonanoyl, 10-(4-Amino-piperidino)-decanoyl; 4-BOC-amino-piperidino-$C_mH_{2m}$-CO- wie 4-BOC-amino-piperidino-carbonyl, 4-BOC-amino-piperidino-acetyl; 4-Dialkylamino-piperidino-$C_mH_{2m}$-CO-wie 4-Dimethylaminopiperidinocarbonyl, 4-Dimethylamino-piperidino-acetyl; 4-Alkoxycarbonylamino-piperidino-$C_mH_{2m}$-CO- wie 4-Ethoxycarbonylamino-piperidino-carbonyl, 4-Methoxycarbonylamino-piperidino-acetyl; 4-Guanidino-piperidino-$C_mH_{2m}$-CO- wie 4-Guanidino-piperidino-carbonyl, 4-Guanidino-piperidinoacetyl; 4-Carboxy-piperidino-$C_mH_{2m}$-CO- wie 4-Carboxy-piperidino-carbonyl, 4-Carboxy-piperidino-acetyl; 4-Alkoxycarbonyl-piperidino-$C_mH_{2m}$-CO- wie 4-Methoxycarbonyl-piperidino-carbonyl, 4-Ethoxycarbonyl-piperidino-carbonyl, 4-Methoxycarbonyl-piperidino-acetyl, 4-Ethoxycarbonyl-piperidino-acetyl; 4-AcNH-piperidino-$C_mH_{2m}$-CO- wie 4-Acetamido-piperidino-carbonyl, 4-Acetamido-piperidino-acetyl; Morpholino-$C_mH_{2m}$-CO- wie Morpholinocarbonyl oder Morpholinoacetyl; 4-BOC-piperazino-$C_mH_{2m}$-CO- wie 4-BOC-piperazinocarbonyl oder 4-BOC-piperazinoacetyl; $H_2N$-C(=NH)-NH-$C_mH_{2m}$-CO- wie Guanidinoacetyl, 3-Guanidino-propionyl, 4-Guanidino-butyryl, 5-Guanidino-pentanoyl, 6-Guanidino-hexanoyl, 7-Guanidino-heptanoyl, 8-Guanidino-octanoyl; HOOC-$C_mH_{2m}$-CO- wie Malonyl, Succinyl, Glutaryl, Adipyl, 6-Carboxyhexanoyl, 7-Carboxyheptanoyl, 8-Carboxy-octanoyl, 9-Carboxynonanoyl, 10-Carboxy-decanoyl, 11-Carboxy-undecanoyl; AOOC-$C_mH_{2m}$-CO- wie Methoxycarbonyl-acetyl, 3-Methoxycarbonyl-propionyl, 4-Methoxycarbonyl-acetyl, 5-Methoxycarbonyl-pentanoyl, 6-Methoxycarbonyl-hexanoyl, 7-Methoxycarbonyl-heptanoyl, 8-Methoxycarbonyl-octanoyl, 9-Methoxycarbonyl-nonanoyl, 10-Methoxycarbonyl-decanoyl, Ethoxycarbonyl-acetyl, 3-Ethoxycarbonyl-propionyl, 4-Ethoxycarbonyl-butyryl, 5-Ethoxycarbonyl-pentanoyl, 6-Ethoxycarbonyl-hexanoyl, 7-Ethoxycarbonyl-heptanoyl, 8-Ethoxycarbonyl-octanoyl, 9-Ethoxycarbonyl-nonanoyl, 10-Ethoxycarbonyl-decanoyl; $H$-$SO_3$-$C_mH_{2m}$-CO- wie Sulfo-acetyl, 3-Sulfo-propionyl, 4-Sulfobutyryl, 5-Sulfo-pentanoyl, 6-Sulfo-hexanoyl, 7-Sulfoheptanoyl, 8-Sulfo-octanoyl, 9-Sulfo-nonanoyl, 10-Sulfo-decanoyl; A-$SO_3$-$C_mH_{2m}$-CO- wie Methoxysulfonyl-acetyl, 3-Methoxysulfonyl-propionyl, 4-Methoxysulfonyl-butyryl, 5-Methoxysulfonyl-pentanoyl, 6-Methoxysulfonyl-hexanoyl, 7-Methoxysulfonyl-heptanoyl, 8-Methoxysulfonyl-octanoyl, 9-Methoxysulfonyl-nonanoyl, 10-Methoxysulfonyl-decanoyl, Ethoxysulfonyl-acetyl, 3-Ethoxysulfonyl-propionyl, 4-Ethoxysulfonyl-butyryl, 5-Ethoxysulfonyl-pentanoyl, 6-Ethoxysulfonyl-hexanoyl, 7-Ethoxysulfonyl-heptanoyl, 8-Ethoxysulfonyl-octanoyl, 9-Ethoxysulfonyl-nonanoyl, 10-Ethoxysulfonyl-decanoyl; $R^5$-$C_mH_{2m}$-O-CO-, vor allem A-O-CO- wie ETOC, IPOC, BOC sowie Ar-$C_mH_{2m}$-O-CO- wie CBZ; $R^5$-$C_mH_{2m}$-CO-, vor allem A-CO- wie Acetyl, Trimethylacetyl oder 3,3-Dimethylbutyryl, aber auch Formyl; $R^5$-$SO_2$- wie A-$SO_2$-, bevorzugt Methylsulfonyl, Isopropylsulfonyl oder tert.-Butylsulfonyl.

Die Verbindungen der Formel I besitzen mindestens zwei chirale Zentren und kommen daher in verschiedenen - optisch-aktiven oder optisch-inaktiven - Formen vor. Die Formel I umschließt alle diese Formen. Die Verbindungen mit 1S-, 2R- und/oder 3S-Konfiguration sind bevorzugt (wobei dem C-Atom, das den Rest $R^4$ trägt, die 1-Stellung, dem C-Atom, das die Reste X-W-$CR^1R^2$-CO-$CH_2CH_2$-CO-NH und $R^3$ trägt, die 3-Stellung zugeordnet ist).

Die oben erwähnten Cycloalkyl- und Phenylgruppen sind vorzugsweise unsubstituiert oder tragen vorzugsweise 1 bis 3, insbesondere 1 oder 2 Substituenten.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ik ausgedrückt werden:

Ia    H-W-$CR^1R^2$-CO-NH-$CH_2CH_2$-CO-NH-$CHR^3$-$(CHOH)_2$-$R^4$;

Ib    $R^6$-O-$C_mH_{2m}$-CO-W-$CR^1R^2$-CO-NH-$CH_2CH_2$-CO-NH-$CHR^3$-$(CHOH)_2$-$R^4$;

Ic    $R^6$-$C_mH_{2m}$-O-CO-W-$CR^1R^2$-CO-NH-$CH_2CH_2$-CO-NH-$CHR^3$-$(CHOH)_2$-$R^4$;

Id    $R^6$-$C_mH_{2m}$-CO-W-$CR^1R^2$-CO-NH-$CH_2CH_2$-CO-NH-$CHR^3$-$(CHOH)_2$-$R^4$;

Ie    $R^7R^8$N-$C_mH_{2m}$-CO-W-$CR^1R^2$-CO-NH-$CH_2CH_2$-CO-NH-$CHR^3$-$(CHOH)_2$-$R^4$;

If    $R^9$-NH-C(=NH)-NH-$C_mH_{2m}$-CO-NH-$CH_2CH_2$-CO-NH-$CHR^3$-$(CHOH)_2$-$R^4$;

Ig    $R^7$OOC-$C_mH_{2m}$-CO-W-$CR^1R^2$-CO-NH-$CH_2CH_2$-CO-NH-$CHR^3$-$(CHOH)_2$-$R^4$;

Ih    $R^7O_3$S-$C_mH_{2m}$-CO-W-$CR^1R^2$-CO-NH-$CH_2CH_2$-CO-NH-$CHR^3$-$(CHOH)_2$-$R^4$;

Ii    $R^7$-O-$(CH_2CH_2O)_n$-$C_mH_{2m}$-CO-NH-$CH_2CH_2$-CO-NH-$CHR^3$-$(CHOH)_2$-$R^4$;

Ij    $R^7R^8$N-CO-W-$CR^1R^2$-CO-NH-$CH_2CH_2$-CO-NH-$CHR^3$-$(CHOH)_2$-$R^4$;

Ik    4-Aminopiperidinocarbonyl-W-$CR^1R^2$-CO-NH-$CH_2CH_2$-CO-NH-$CHR^3$-$(CHOH)_2$-$R^4$.

Insbesondere bevorzugt sind Verbindungen der Teilformeln:

(a) Iaa bis Ika, die den Formeln Ia bis Ik entsprechen, worin jedoch zusätzlich

-W-$CR^1R^2$-CO- Phe, Pla, Mal oder -$CH_2$-CH-

$(CH_2C_6H_5)$-CO-
bedeutet;

(b) Iab bis Ikb sowie Iaab bis Ikab, die den Formeln Ia bis Ik sowie Iaa bis Ika entsprechen, worin jedoch zusätzlich $R^3$ Cyclohexylmethyl bedeutet;

(c) Iac bis Ikc, Iaac bis Ikac sowie Iabc bis Ikbc, die den Formeln Ia bis Ik, Iaa bis Ika sowie Iab bis Ikb entsprechen, worin jedoch zusätzlich $R^4$ A oder Cycloalkyl mit 3-6 C-Atomen bedeutet.

Insbesondere sind bevorzugt Verbindungen der Teilformeln:

I* sowie Ia* bis Ik*, die den Formeln I sowie Ia bis Ik sowie solche Verbindungen, die den anderen vorstehend genannten Teilformeln entsprechen, worin jedoch zusätzlich

$R^4$     Cyclopropyl bedeutet;

I' sowie Ia' bis Ik', die den Formeln I sowie Ia bis Ik sowie solche Verbindungen, die den anderen vorstehend genannten Teilformeln entsprechen, worin jedoch zusätzlich

$R^4$     Isobutyl bedeutet.

Eine besonders bevorzugte Gruppe von Verbindungen entspricht der Formel I,
worin

X

4-BOC-aminopiperidinocarbonyl oder 4-Aminopiperidinocarbonyl,

-W-CR$^1$R$^2$-CO-

Phe,

$R^3$

Cyclohexylmethyl und

$R^4$

Isobutyl oder Cyclopropyl bedeuten.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A-45665, EP-A-77028, EP-A-77029, EP-A-81783, EP-A-249096, EP-A-332008) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B. solche die der Formel I entsprechen, aber an Stelle einer His-Gruppe eine N(im)-R'-His-Gruppe (worin R' eine Aminoschutzgruppe bedeutet, z.B. BOM oder DNP) enthalten.

Ferner sind Ausgangsstoffe bevorzugt, die an Stelle des H-Atoms einer Hydroxygruppe eine Hydroxyschutzgruppe tragen, z.B. solche der Formel X-W-CR$^1$R$^2$-CO-NH-CH$_2$CH$_2$-CO-NH-CHR$^3$-(CHOR'')$_2$-R$^4$, worin R'' eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. DNP), Aralkoxymethyl- (z.B. BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, IPOC, BOC, 2-Jodethoxycarbonyl; Aralkyloxycarbonyl wie CBZ, 4-Methoxybenzyloxycarbonyl, FMOC. Bevorzugte Aminoschutzgruppen sind BOC, DNP and BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich.

Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°; vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Dioxan bei 15-30° abgespalten weden, die FMOC-Gruppe mit einer etwa 5-20%igen Lösung von Dimethylamin, Diethylamin oder Piperidin in DMF bei 15-30°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können auch durch direkte Kondensation (Peptidsynthese) aus einer Carbonsäure- (Formel II) und einer Hydroxy- bzw. Aminokomponente (Formel III) erhalten werden. Als Carbonsäurekomponenten eignen sich z.B. solche der Teilformeln (a) X-OH, (b) X-W-CR$^1$R$^2$-COOH oder (c) X-W-CR$^1$R$^2$-CO-NH-CH$_2$CH$_2$-CO-OH, als Hydroxy- bzw. Aminokomponenten solche der Teilformeln (a) HW-CR$^1$R$^2$-CO-NH-CH$_2$CH$_2$-CO-NH-CHR$^3$-(CHOH)$_2$-R$^4$, (b) H-NH-CH$_2$CH$_2$-CO-NH-CHR$^3$-(CHOH)$_2$-R$^4$ oder (c) H$_2$N-CHR$^3$-(CHOH)$_2$-R$^4$. Dabei arbeitet man zweckmäßig nach üblichen Methoden der Peptid-Synthese, wie sie z.B. in Houben-Weyl, 1.c., Band 15/II, Seiten 1-806 (1974) beschrieben sind; diese Methoden können, falls W = 0 ist, auch auf die Kondensation gemäß (a) übertragen werden, wobei eine Esterbindung entsteht.

Die Reaktion gelingt vorzugsweise in Gegenwart eines Dehydratisierungsmittels, z.B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blokkiert sind. Die Säurederivate II können z.B. in Form ihrer aktivierten Ester verwendet werden, die zweckmäßig in situ gebildet werden, z.B. durch Zusatz von HOBt oder N-Hydroxysuccinimid.

Die Ausgangsstoffe der Formeln II und III sind großenteils bekannt. Sofern sie nicht bekannt sind, können sie nach bekannten Methoden, z.B. den oben angegebenen Methoden der Kondensation und der Abspaltung von Schutzgruppen hergestellt werden.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann z. B. eine Verbindung der Formel I, die eine $R^9$-$C_xH_{2x}$-O-CO-NH-, eine AcNH- oder eine AOOC-Gruppe enthält, in die entsprechende Verbindung der Formel I umgewandelt werden, die stattdessen eine $H_2N$- oder eine HOOC-Gruppe enthält, zweckmäßig durch selektive Solvolyse nach einer der oben angegebenen Methoden. AOOC-Gruppen können z.B. mit NaOH oder KOH in Wasser-Dioxan bei Temperaturen zwischen 0 und 40°, vorzugsweise 10 und 30°, verseift werden.

Es ist auch möglich, eine Verbindung der Formel I, die eine freie primäre oder sekundäre Aminogruppe enthält, zu acylieren. So kann man insbesondere Verbindungen der Formel I, in denen X H bedeutet, mit acylierenden Mitteln der Formel X-Cl oder X-Br (worin X von H verschieden ist) umsetzen, zweckmäßig in Gegenwart eines inerten Lösungsmittels wie THF und/oder einer Base wie Pyridin oder Triethylamin bei Temperaturen zwischen -10 und +30°.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Trifluoressigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Application Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-332008 beschriebenen Verbindungen verabreicht, vorzugsweise in Dosierungen zwischen etwa 10 mg und 1 g, insbesondere zwischen 50 und 500 mg pro Dosierungseinheit. Die tägliche Dosierung liegt vorzugsweise zwischen etwa 0,2 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt. Renin-abhängige Hypertension und Hyperaldosteronismus können wirksam behandelt werden durch Verabfolgung von Dosierungen zwischen insbesondere etwa 0,2 und 20, vorzugsweise zwischen 1 und 10 mg/kg Körpergewicht. Für diagnostische Zwecke können die neuen Verbindungen zweckmäßig in Einzeldosen zwischen etwa 0,1 und 10 mg/kg Körpergewicht verabreicht werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation. FAB = Massenspektrum nach der "Fast-Atom-Bombardment"-Methode.

Beispiel 1

Man löst 1 g 3S-[tert.-Butoxycarbonyl-L-(N-imi-benzyloxymethyl-histidyl)-β-alanyl-amino]-4-cyclohexyl-1-cyclopropylbutan-1S,2R-diol [= "3S-BOC-(imi-BOM-His)-βAla-amino-4-cyclohexyl-1-cyclopropyl-butan-1S,2R-diol"; erhältlich durch Reaktion von N-BOC-3-cyclohexyl-L-alaninal mit Trimethylsilylcyanid/KCN/18-Krone-6 zu 3S-BOC-amino-4-cyclohexyl-2-hydroxy-butyronitril (Gemisch von Diastereomeren), Hydrolyse mit HCl/CH$_3$COOH zu 3S-Amino-4-cyclohexyl-2-hydroxy-buttersäure (Gemisch), Veresterung und nachfolgende Reaktion mit (BOC)$_2$O zu 3S-BOC-amino-4-cyclohexyl-2-hydroxy-buttersäure-methylester, Reaktion mit 2,2-Dimethoxypropan zu 3-BOC-4S-cyclohexylmethyl-2,2-dimethyl-oxazolidin-5R-carbonsäure-methylester (FAB: M + 1 = 356), Reduktion mit Diisobutylaluminiumhydrid in Toluol zu 3-BOC-4S-cyclohexylmethyl-5R-formyl-2,2-dimethyl-oxazolidin, Umsetzung mit Cyclopropylmagnesiumbromid und nachfolgende Hydrolyse zu 3-BOC-4S-cyclohexylmethyl-5R-(1-cyclopropyl-1-hydroxymethyl)-2,2-dimethyl-oxazolidin (Gemisch; FAB: M + 1 = 368), Ringspaltung mit 4 n HCl/Dioxan zu 3S-Amino-4-cyclohexyl-1-cyclopropyl-butan-1S,2R-diol ("IIIa") und Kondensation mit BOC-(imi-BOM-His)-βAla-OH] in 30 ml Ethanol, hydriert an 0,3 g 10%ig. Pd-C bei 20° und 1 bar bis zum Stillstand der H$_2$-Aufnahme, filtriert, dampft ein und erhält nach chromatographischer Reinigung an Kieselgel 3S-BOC-His-βAla-amino-4-cyclohexyl-1-cyclopropyl-butan-1S,2R-diol.

Beispiel 2

Analog Beispiel 1 erhält man durch Hydrogenolyse von 3S-(4-Benzyloxycarbonyl-4-CBZ-amino-butyryl-Phe-βAla-amino)-4-cyclohexyl-1-cyclopropyl-butan-1S,2R-diol (erhältlich durch Kondensation von 4-Benzyloxycarbonyl-4-CBZ-aminobutyryl-Phe-βAla-OH mit IIIa) das 3S-(4-Amino-4-carboxy-butyryl-Phe-βAla-amino)-4-cyclohexyl-1-cyclopropyl-butan-1S,2R-diol.

Analog erhält man durch Hydrogenolyse der entsprechenden Benzyloxycarbonyl-CBZ-amino-derivate:
3S-(3-Amino-3-carboxy-propionyl-Phe-βAla-amino)-4-cyclohexyl-1-cyclopropyl-butan-1S,2R-diol
3S-[N-(3-Amino-3-carboxy-propyl)-carbamoyl-Phe-βAla-amino]-4-cyclohexyl-1-cyclopropyl-butan-1S,2R-diol
3S-[N-(4-Amino-4-carboxy-butyl)-carbamoyl-Phe-βAla-amino]-4-cyclohexyl-1-cyclopropyl-butan-1S,2R-diol.

Beispiel 3

Ein Gemisch von 1 mmol 3S-BOC-(imi-DNP-His)-βAla-amino-4-cyclohexyl-1-cyclopropyl-butan-1S,2R-diol [erhältlich durch Kondensation von BOC-(imi-DNP-His)-βAla-OH mit IIIa], 2 g 2-Mercaptoethanol, 20 ml DMF und 20 ml Wasser wird unter Rühren bei 20° mit wässeriger Na$_2$CO$_3$-Lösung auf pH 8 eingestellt und noch 2 Std. bei 20° gerührt. Nach üblicher Aufarbeitung erhält man 3S-BOC-His-βAla-amino-4-cyclohexyl-1-cyclopropyl-butan-1S,2R-diol.

Beispiel 4

Eine Lösung von 2,27 g IIIa in 60 ml Dichlormethan wird mit 1,01 g N-Methylmorpholin versetzt. Unter Rühren gibt man 4,5 g 4-BOC-amino-piperidino-carbonyl-Phe-βAla-OH, 1,35 g HOBt und eine Lösung von 2,06 g DCCI in 50 ml Dichlormethan hinzu, rührt 12 Std. bei 0-5°, filtriert den ausgeschiedenen Dicyclohexylharnstoff ab und dampft das Filtrat ein. Nach üblicher Aufarbeitung erhält man 3S-(4-BOC-amino-piperidinocarbonyl-Phe-βAla-amino)-4-cyclohexyl-1-cyclopropyl-butan-1S,2R-diol, F. 107-108°; FAB: M + 1 = 672.

Beispiel 5

Analog Beispiel 4 erhält man aus IIIa
mit BOC-Phe-βAla-OH das 3S-(BOC-Phe-βAla-amino)-4-cyclohexyl-1-cyclopropyl-butan-1S,2R-diol;
mit 4-Hydroxy-piperidinocarbonyl-Phe-βAla-OH das 3S-(4-Hydroxy-piperidinocarbonyl-Phe-βAla-amino)-4-cyclohexyl-1-cyclopropyl-butan-1S,2R-diol;
mit 4-Dimethylamino-piperidinocarbonyl-Phe-βAla-OH das 3S-(4-Dimethylamino-piperidinocarbonyl-Phe-βAla-amino)-4-cyclohexyl-1-cyclopropyl-butan-1S,2R-diol;

mit Morpholinocarbonyl-Phe-βAla-OH das 3S-(Morpholinocarbonyl-Phe-βAla-amino)-4-cyclohexyl-1-cyclopropyl-butan-1S,2R-diol;

mit 4-Ethoxycarbonyl-piperidinocarbonyl-Phe-βAla-OH das 3S-(4-Ethoxycarbonyl-piperidinocarbonyl-Phe-βAla-amino)-4-cyclohexyl-1-cyclopropyl-butan-1S,2R-diol;

mit Methylsulfonyl-Phe-βAla-OH das 3S-(Methylsulfonyl-Phe-βAla-amino)-4-cyclohexyl-1-cyclopropyl-butan-1S,2R-diol;

mit Isopropylsulfonyl-Phe-βAla-OH das 3S-(Isopropylsulfonyl-Phe-βAla-amino)-4-cyclohexyl-1-cyclopropyl-butan-1S,2R-diol;

mit 3-BOC-amino-3-methyl-butyryl-Phe-βAla-OH das 3S-(3-BOC-amino-3-methyl-butyryl-Phe-βAla-amino)-4-cyclohexyl-1-cyclopropyl-butan-1S,2R-diol;

mit 6-BOC-amino-hexanoyl-Phe-βAla-OH das 3S-(6-BOC-aminohexanoyl-Phe-βAla-amino)-4-cyclohexyl-1-cyclopropyl-butan-1S,2R-diol;

mit 3,6,8-Trioxa-nonanoyl-Phe-βAla-OH das 3S-(3,6,8-Trioxanonanoyl-Phe-βAla-amino)-4-cyclohexyl-1-cyclopropyl-butan-1S,2R-diol;

mit 6-Methoxycarbonyl-hexanoyl-Phe-βAla-OH das 3S-(6-Methoxycarbonyl-hexanoyl-Phe-βAla-amino)-4-cyclohexyl-1-cyclopropyl-butan-1S,2R-diol;

mit N-Ethyl-carbamoyl-Phe-βAla-OH das 3S-(N-Ethyl-carbamoyl-Phe-βAla-amino)-4-cyclohexyl-1-cyclopropyl-butan-1S,2R-diol;

mit N-BOC-carbamoyl-Phe-βAla-OH das 3S-(N-BOC-carbamoyl-Phe-βAla-amino)-4-cyclohexyl-1-cyclopropyl-butan-1S,2R-diol;

mit N-(5-Dimethylaminopentyl)-carbamoyl-Phe-βAla-OH das 3S-[N-(5-Dimethylaminopentyl)-carbamoyl-Phe-βAla-amino]-4-cyclohexyl-1-cyclopropyl-butan-1S,2R-diol;

mit Morpholinocarbonyl-Pla-βAla-OH das 3S-(Morpholinocarbonyl-Pla-βAla-amino)-4-cyclohexyl-1-cyclopropyl-butan-1S,2R-diol;

mit 4-BOC-amino-piperidinocarbonyl-Pla-βAla-OH das 3S-(4-BOC-amino-piperidinocarbonyl-Pla-βAla-amino)-4-cyclohexyl-1-cyclopropyl-butan-1S,2R-diol;

mit 2-Benzyl-3-tert.-butylsulfonyl-propionyl-βAla-OH das 3S-(2-Benzyl-3-tert.-butylsulfonyl-propionyl-βAla-amino)-4-cyclohexyl-1-cyclopropyl-butan-1S,2R-diol;

mit 4-BOC-amino-piperidinocarbonyl-Z-βAla-OH (Z = Ada, Cal, Leu, Mal αNal, βNal, Nle, NVA, 2-Tia, 2-Tiz oder Trp):

3S-(4-BOC-amino-piperidinocarbonyl-Ada-βAla-amino)-4-cyclohexyl-1-cyclopropyl-butan-1S,2R-diol;

3S-(4-BOC-amino-piperidinocarbonyl-Cal-βAla-amino)-4-cyclohexyl-1-cyclopropyl-butan-1S,2R-diol;

3S-(4-BOC-amino-piperidinocarbonyl-Leu-βAla-amino)-4-cyclohexyl-1-cyclopropyl-butan-1S,2R-diol;

3S-(4-BOC-amino-piperidinocarbonyl-Mal-βAla-amino)-4-cyclohexyl-1-cyclopropyl-butan-1S,2R-diol;

3S-(4-BOC-amino-piperidinocarbonyl-αNal-βAla-amino)-4-cyclohexyl-1-cyclopropyl-butan-1S,2R-diol;

3S-(4-BOC-amino-piperidinocarbonyl-βNal-βAla-amino)-4-cyclohexyl-1-cyclopropyl-butan-1S,2R-diol;

3S-(4-BOC-amino-piperidinocarbonyl-Nle-βAla-amino)-4-cyclohexyl-1-cyclopropyl-butan-1S,2R-diol;

3S-(4-BOC-amino-piperidinocarbonyl-Nva-βAla-amino)-4-cyclohexyl-1-cyclopropyl-butan-1S,2R-diol;

32-[4-BOC-amino-piperidinocarbonyl-(2-Tia)-βAla-amino]-4-cyclohexyl-1-cyclopropyl-butan-1S,2R-diol;

3S-[4-BOC-amino-piperidinocarbonyl-(2-Tiz)-βAla-amino]-4-cyclohexyl-1-cyclopropyl-butan-1S,2R-diol;

3S-(4-BOC-amino-piperidinocarbonyl-Trp-βAla-amino)-4-cyclohexyl-1-cyclopropyl-butan-1S,2R-diol;

mit H-Pla-βAla-OH das 3S-(H-Pla-βAla-amino)-4-cyclohexyl-1-cyclopropyl-butan-1S,2R-diol.

Beispiel 6

Analog Beispiel 4 erhält man aus 4-BOC-aminopiperidinocarbonyl-Phe-βAla-OH oder 4-Hydroxy-piperidinocarbonyl-Phe-βAla-OH:

mit 2S-Amino-1-cyclohexyl-6-methyl-heptan-3R,4S-diol:

2S-(4-BOC-amino-piperidinocarbonyl-Phe-βAla-amino)-1-cyclohexyl-6-methyl-heptan-3R,4S-diol, F. 91-92°; FAB: M + 1 = 688

2S-(4-Hydroxy-piperidinocarbonyl-Phe-βAla-amino)-1-cyclohexyl-6-methyl-heptan-3R,4S-diol;

mit 2S-Amino-1-cyclohexyl-octan-3R,4S-diol:

2S-(4-BOC-amino-piperidinocarbonyl-Phe-βAla-amino)-1-cyclohexyl-octan-3R,4S-diol

2S-(4-Hydroxy-piperidinocarbonyl-Phe-βAla-amino)-1-cyclohexyl-octan-3R,4S-diol;

mit 2S-Amino-1-cyclohexyl-hexan-3R,4S-diol:

2S-(4-BOC-amino-piperidinocarbonyl-Phe-βAla-amino)-1-cyclohexyl-hexan-3R,4S-diol

2S-(4-Hydroxy-piperidinocarbonyl-Phe-βAla-amino)-1-cyclohexyl-hexan-3R,4S-diol;

mit 3S-Amino-4-cyclohexyl-1-cyclopentyl-butan-1S,2R-diol:

3S-(4-BOC-amino-piperidinocarbonyl-Phe-βAla-amino)-4-cyclohexyl-1-cyclopentyl-butan-1S,2R-diol

3S-(4-Hydroxy-piperidinocarbonyl-Phe-βAla-amino)-4-cyclohexyl-1-cyclopentyl-butan-1S,2R-diol.

## Beispiel 7

Analog Beispiel 4 erhält man aus 4-BOC-amino-piperidinocarbonyl-Phe-OH und 3S-(H-βAla-amino)-4-cyclohexyl-1-cyclopropyl-butan-1S,2R-diol das 3S-(4-BOC-amino-piperidinocarbonyl-Phe-βAla-amino)-4-cyclohexyl-1-cyclopropyl-butan-1S,2R-diol, F. 107-108°.

## Beispiel 8

Analog Beispiel 4 erhält man aus 6-Ethoxysulfonyl-hexansäure und 3S-(H-Phe-βAla-amino)-1,4-bis-cyclohexyl-butan-1S,2R-diol das 3S-(6-Ethoxysulfonyl-hexanoyl-Phe-βAla-amino)-1,4-bis-cyclohexyl-butan-1S,2R-diol.

## Beispiel 9

Eine Lösung von 1 g 2S-(4-BOC-amino-piperidinocarbonyl-Phe-βAla-amino)-1-cyclohexyl-6-methyl-heptan-3R,4S-diol in 20 ml Dichlormethan und 20 ml Trifluoressigsäure wird 1 Std. bei 20° gerührt und dann eingedampft. Man erhält 2S-(4-Amino-piperidinocarbonyl-Phe-βAla-amino)-1-cyclohexyl-6-methyl-heptan-3R,4S-diol, Trifluoracetat, F. 140-141°; 1/3-Citrat, F. 183-184°; FAB: M + 1 = 588.

Analog erhält man durch Spaltung der entsprechenden BOC-amino-derivate mit Trifluoressigsäure:

3S-(4-Amino-piperidinocarbonyl-Phe-βAla-amino)-4-cyclohexyl-1-cyclopropyl-butan-1S,2R-diol, Trifluoracetat, FAB: M + 1 = 572
3S-(H-Phe-βAla-amino)-4-cyclohexyl-1-cyclopropyl-butan-1S,2R-diol
3S-(3-Amino-3-methyl-butyryl-Phe-βAla-amino)-4-cyclohexyl-1-cyclopropyl-butan-1S,2R-diol
3S-(6-Amino-hexanoyl-Phe-βAla-amino)-4-cyclohexyl-1-cyclopropyl-butan-1S,2R-diol
3S-(Carbamoyl-Phe-βAla-amino)-4-cyclohexyl-1-cyclopropylbutan-1S,2R-diol
3S-(4-Amino-piperidinocarbonyl-Pla-βAla-amino)-4-cyclohexyl-1-cyclopropyl-butan-1S,2R-diol
3S-(4-Amino-piperidinocarbonyl-Ada-βAla-amino)-4-cyclohexyl-1-cyclopropyl-butan-1S,2R-diol
3S-(4-Amino-piperidinocarbonyl-Cal-βAla-amino)-4-cyclohexyl-1-cyclopropyl-butan-1S,2R-diol
3S-(4-Amino-piperidinocarbonyl-Leu-βAla-amino)-4-cyclohexyl-1-cyclopropyl-butan-1S,2R-diol
3S-(4-Amino-piperidinocarbonyl-Mal-βAla-amino)-4-cyclohexyl-1-cyclopropyl-butan-1S,2R-diol
3S-(4-Amino-piperidinocarbonyl-αNal-βAla-amino)-4-cyclohexyl-1-cyclopropyl-butan-1S,2R-diol
3S-(4-Amino-piperidinocarbonyl-βNal-βAla-amino)-4-cyclohexyl-1-cyclopropyl-butan-1S,2R-diol

3S-(4-Amino-piperidinocarbonyl-Nle-βAla-amino)-4-cyclohexyl-1-cyclopropyl-butan-1S,2R-diol
3S-(4-Amino-piperidinocarbonyl-Nva-βAla-amino)-4-cyclohexyl-1-cyclopropyl-butan-1S,2R-diol
3S-[4-Amino-piperidinocarbonyl-(2-Tia)-βAla-amino]-4-cyclohexyl-1-cyclopropyl-butan-1S,2R-diol
3S-[4-Amino-piperidinocarbonyl-(2-Tiz)-βAla-amino]-4-cyclohexyl-1-cyclopropyl-butan-1S,2R-diol
3S-(4-Amino-piperidinocarbonyl-Trp-βAla-amino)-4-cyclohexyl-1-cyclopropyl-butan-1S,2R-diol
2S-(4-Amino-piperidinocarbonyl-Phe-βAla-amino)-1-cyclohexyloctan-3R,4S-diol
3S-(4-Amino-piperidinocarbonyl-Phe-βAla-amino)-4-cyclohexyl-1-cyclopentyl-butan-1S,2R-diol
2S-(4-Amino-piperidinocarbonyl-Phe-βAla-amino)-1-cyclohexylhexan-3R,4S-diol.

## Beispiel 10

Ein Gemisch von 1 g 3S-(4-Ethoxycarbonyl-piperidinocarbonyl-Phe-βAla-amino)-4-cyclohexyl-1-cyclopropyl-butan-1S,2R-diol, 50 ml Dioxan und 20 ml 2 n wässeriger NaOH-Lösung wird 3 Std. bei 20° gerührt. Man arbeitet wie üblich auf und erhält 3S-(4-Carboxy-piperidinocarbonyl-Phe-βAla-amino)-4-cyclohexyl-1-cyclopropyl-butan-1S,2R-diol.

Analog erhält man durch Hydrolyse der entsprechenden Methyl- oder Ethylester:
3S-(6-Carboxy-hexanoyl-Phe-βAla-amino)-4-cyclohexyl-1-cyclopropyl-butan-1S,2R-diol.

## Beispiel 11

Analog Beispiel 1 erhält man durch Hydrogenolyse von 3S-(CBZ-Phe-βAla-amino)-4-cyclohexyl-1-cyclopropyl-butan-1S,2R-diol das 3S-(H-Phe-βAla-amino)-4-cyclohexyl-1-cyclopropyl-butan-1S,2R-diol.

## Beispiel 12

(a) Man löst 455 mg 3S-(H-Phe-βAla-amino)-4-cyclohexyl-1-cyclopropyl-butan-1S,2R-diol in 25 ml THF. Unter Rühren wird eine Lösung von 117 g $CH_3SO_2Cl$ in 3 ml THF hinzugetropft. Man rührt noch 3 Std. bei 20°, arbeitet wie üblich auf und erhält 3S-(Methansulfonyl-Phe-βAla-amino)-4-cyclohexyl-1-cyclopropyl-butan-1S,2R-diol.

(b) Analog erhält man mit Acetylchlorid das 3S-(Acetyl-Phe-βAla-amino)-4-cyclohexyl-1-cyclopropyl-butan-1S,2R-diol.

Die nachfolgenden Beispiele betreffen pharmazeutische Zubereitungen.

## Beispiel A: Tabletten

Ein Gemisch von 1 kg 3S-(4-Amino-piperidinocarbonyl-Phe-βAla-amino)-4-cyclohexyl-1-cyclopropyl-butan-1S,2R-dioltrifluoracetat, 4 kg Lactose, 1,2 kg Maisstärke, 200 g Talk und 100 g Magnesiumstearat wird in üblicher Weise zu Tabletten verpreßt, derart, daß jede Tablette 100 mg Wirkstoff enthält.

**Beispiel B: Dragees**

Analog Beispiel A werden Tabletten gepreßt, die anschließend in üblicher Weise mit einem Überzug aus Saccharose, Maisstärke, Talk, Tragant und Farbstoff überzogen werden.

**Beispiel C: Kapseln**

500 g 2S-(4-Amino-piperidinocarbonyl-Phe-βAla-amino)-1-cyclohexyl-6-methyl-heptan-3R,4S-diol-1/3-citrat werden in üblicher Weise in Hartgelatinekapseln gefüllt, so daß jede Kapsel 500 mg Wirkstoff enthält.

**Beispiel D: Injektionsgläser**

Eine Lösung von 100 g 3S-(4-Amino-piperidinocarbonyl-Phe-βAla-amino)-4-cyclohexyl-1-cyclopropyl-butan-1S,2R-dioltrifluoracetat in 4 l zweifach destilliertem Wasser wird mit 2 n Salzsäure auf pH 6,5 eingestellt, steril filtriert und in Injektionsgläser abgefüllt. Man lyophilisiert unter sterilen Bedingungen und verschließt steril. Jedes Injektionsglas enthält 100 mg Wirkstoff.

**Beispiel E: Suppositorien**

Man schmilzt ein Gemisch von 50 g 3S-(4-BOC-aminopiperidinocarbonyl-Phe-βAla-amino)-4-cyclohexyl-1-cyclopropyl-butan-1S,2R-diol mit 10 g Sojalecithin und 140 g Kakaobutter, gießt in Formen und läßt erhalten. Jedes Suppositorium enthält 250 mg Wirkstoff.

**Patentansprüche**

1. Aminosäurederivate der Formel I

$$X-W-CR^1R^2-CO-NH-CH_2CH_2-CO-NH-CHR^3-(CHOH)_2-R^4 \quad I$$

worin

X

H, $R^5$-O-$C_mH_{2m}$-CO-, $R^5$-$C_mH_{2m}$-O-CO-, $R^5$-$C_mH_{2m}$-CO-, $R^5$-$SO_2$-, $R^6R^7$N-$C_mH_{2m}$-CO-, $R^6$-NH-C(=NH)-NH-$C_mH_{2m}$-CO-, $R^6$OOC-$C_mH_{2m}$-CO-, $R^6O_3S$-$C_mH_{2m}$-CO-, $R^6$-O-$(CH_2CH_2O)_n$-$C_mH_{2m}$-CO-, $A_3N^+$-$C_mH_{2m}$-CO-An$^-$ oder $R^6$OOC-CH($NR^8R^9$)-$C_pH_{2p}$-Y-CO-,

W und Y

jeweils $CH_2$, O oder NH,

$R^1$, $R^7$ und $R^8$

jeweils H oder A,

$R^2$, $R^3$ und $R^5$

jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^4$

H, A, Ar-alkyl, Cycloalkyl mit 3-7 C-Atomen oder Cycloalkylalkyl mit 4-11 C-Atomen,

$R^6$ und $R^9$

jeweils H, A oder $R^5$-$C_pH_{2p}$-Y-CO-,

$R^6R^7$N

auch eine unsubstituierte oder eine durch A, OH, $NH_2$, NHA, $NA_2$, NHAc, NH-CO-$C_xH_{2x}$-O-$R^{10}$, NH-CO-O-$C_xH_{2x}$-$R^{10}$, Hydroxy-alkyl, COOH, COOA, $CONH_2$, Amino-alkyl, HAN-alkyl, $A_2$N-alkyl, $A_3N^⊕$alkyl An$^⊖$, NH-CO-$NH_2$, NH-CO-NHA, Guanidinyl oder Guanidinyl-alkyl substituierte Pyrrolidino-, Piperidino-, Morpholino- oder Piperazino-gruppe,

$R^{10}$

H, A, Ar-alkyl oder CN,

m, p und x

jeweils 0, 1, 2, 3, 4, 5, 6, 7, 8, 9 oder 10,

n

0, 1, 2 oder 3,

Ar

unsubstituiertes oder ein- oder mehrfach durch A, OA, Hal, $CF_3$, OH, $NO_2$, Hydroxy-alkyl, $NH_2$, NHA, $NA_2$, NHAc, NH-$SO_2$-A, SA, SO-A, $SO_2$-A, $SO_2NH_2$, $SO_2NHA$, COOH, COOA, $CONH_2$, CN, Amino-alkyl, HAN-alkyl, $A_2$N-alkyl, $A_3N^⊕$-alkyl An$^⊖$ und/oder Guanidinyl-alkyl substituiertes Phenyl oder unsubstituiertes Naphthyl,

Het

einen gesättigten oder ungesättigten 5-oder 6-gliedrigen heterocyclischen Rest mit 1-4 N-, O- und/oder S-Atomen, der mit einem Benzolring kondensiert und/oder ein- oder mehrfach durch A, OA, Hal, $CF_3$, OH, $NO_2$, Carbonylsauerstoff, $NH_2$, NHA, $NA_2$, NHAc, NH-COOA, NHCOOAr, NHCOOCH$_2$Ar, NH-$SO_2$-A, SA, SO-A, $SO_2$-A, $SO_2NH_2$, $SO_2NHA$, COOH, COOA, $CONH_2$, CN, Ar, Ar-alkyl, Ar-alkenyl, Hydroxy-alkyl, Amino-alkyl, HAN-alkyl, $A_2$N-alkyl und/oder $A_3N^⊕$-alkyl An$^⊖$ substituiert sein kann und/oder dessen N- und/oder S-Heteroatome auch oxydiert sein können,

Hal

F, Cl, Br oder J,

Ac

A-CO-, Ar-CO-, Ar-alkyl-CO- oder A-NH-CO-,

$An^{\ominus}$

ein Anion, das auch fehlen kann, wenn stattdessen eine in der Verbindung der Formel I enthaltene Carboxygruppe in Form eines Carboxylatanions vorliegt,

-alkyl

eine Alkylengruppe mit 1-8 C-Atomen und

A

Alkyl mit 1-8 C-Atomen bedeuten, worin ferner an Stelle einer oder mehrerer -NH-CO-Gruppen auch eine oder mehrere -NA-CO-Gruppen stehen können,

sowie deren Salze.

2.

a) 2S-(4-Amino-piperidinocarbonyl-Phe-$\beta$Ala-amino)-1-cyclohexyl-6-methyl-heptan-3R,4S-diol;
b) 3S-(4-Amino-piperidinocarbonyl-Phe-$\beta$Ala-amino)-4-cyclohexyl-1-cyclopropyl-butan-1S,2R-diol.

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Carbonsäure der Formel II

$X-G^1-OH$     II

worin
$G^1$

(a) fehlt,
(b) $-W-CR^1R^2-CO-$,
(c)     $-W-CR^1R^2-CO-NH-CH_2CH_2-CO-$
bedeutet

oder eines ihrer reaktionsfähigen Derivate mit einer Verbindung der Formel III

$H-G^2-NH-CHR^3-(CHOH)_2-R^4$     III

worin
$G^2$

(a)     $-W-CR^1R^2-CO-NH-CH_2CH_2-CO-$,
(b) $-NH-CH_2CH_2-CO-$ bedeutet,
(c) fehlt

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder eine freie Aminogruppe durch Behandeln mit einem acylierenden Mittel acyliert und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung der reninabhängigen Hypertension oder des Hyperaldosteronismus.